# EUROPÄISCHE PATENTSCHRIFT

(11) **EP 1 090 074 B1**
(45) Veröffentlichungstag und Bekanntmachung des Hinweises auf die Patenterteilung: **17.05.2006**
(21) Anmeldenummer: 99926327.0
(22) Anmeldetag: 18.05.1999
(51) Int. Cl.: C09C 1/00, C09D 5/36, C09D 11/02, C08K 9/02, A61K 8/00

(54) **PIGMENTMISCHUNG**
PIGMENT MIXTURE
MELANGE PIGMENTAIRE

(30) Priorität: 28.05.1998 DE 19823866
(43) Veröffentlichungstag der Anmeldung: 11.04.2001
(73) Patentinhaber: Merck Patent GmbH, 64293 Darmstadt (DE)
(72) Erfinder: VOGT, Reiner, D-64289 Kranichstein (DE); SCHOEN, Sabine, D-64287 Darmstadt (DE); SCHÜL, Norbert, D-64646 Heppenheim (DE); OSTERRIED, Karl, D-64807 Dieburg (DE); MUNZ, Johann, D-68649 Gro -Rohrheim (DE)
(86) Internationale Anmeldenummer: PCT/EP1999/003423
(87) Internationale Veröffentlichungsnummer: WO 1999/061529

(56) Entgegenhaltungen:
- EP-A- 0 562 329
- DE-A- 4 240 511
- DE-A- 19 614 636
- DE-A- 19 614 637

## Beschreibung

Die vorliegende Erfindung betrifft Pigmentmischungen bestehend aus mindestens zwei Komponenten, wobei Komponente A mit ein oder mehreren Metalloxiden und/oder Metallen beschichtete SiO₂-Flakes und Komponente B ein oder mehrere beschichtete oder unbeschichtete Effektpigmente sind, sowie deren Verwendung in Lacken, Farben, Druckfarben, Kunststoffen und kosmetischen Formulierungen.

Deckvermögen und Glanz sind bei plättchenförmigen Pigmenten oftmals nur schwer gleichzeitig in befriedigendem Ausmaß zu realisieren. So zeichnen sich etwa mit einer oder mehreren dünnen Metalloxidschichten belegte Glimmerplättchen oder SiO₂-Flakes durch Interferenzfarben und einen hohen Glanz, gleichzeitig aber auch wegen des durchsichtigen Substrats durch eine hohe Transparenz und damit ein vergleichsweises geringes Deckvermögen, aus.

So wird in der EP 0 562 329 ein Pigmentgemisch beansprucht enthaltend eisenoxidbeschichtete Aluminiumflakes in Kombination mit eisenoxidbeschichteten Glimmerpigmenten.

Aus der DE-A-42 40 511 ist eine Pigmentmischung bekannt, die aus einem Interferenzpigment und einem plättchenförmigen Farbpigment besteht. Das Interferenzpigment sind mit Metalloxiden beschichtete Glimmer- oder SiO₂-Flakes und das Farbpigment können farbige, unbeschichtete SiO₂-Flakes sein. Dieses Pigmentgemisch wird in Lacke, Druckfarben oder Kunststoffen eingearbeitet.

Aus der DE 19614636 A1 und DE 19614637 A1 sind Glanzpigmente auf Basis von mit reduziertem Titandioxidbeschichteten SiO₂-Plättchen bzw. goniochromatische Pigmente, auf der Basis von beschichteten SiO₂₋Plättchen bekannt, die auch in Abmischung mit transparenten und deckenden Weiß-, Bunt- und Schwarzpigmenten sowie Glanzpigmenten auf der Basis von metalloxidbeschichteten Glimmer- und Metallpigmenten, plättchenförmigen Eisenoxiden, Graphit, Molybdänsulfid und plättchenförmigen organischen Pigmenten verwendet werden können.

Aufgabe der vorliegenden Erfindung ist es eine Pigmentmischung bereitzustellen, die sich durch ein relativ hohes Deckvermögen auszeichnet und sich gut in das jeweilige Anwendungssystem einarbeiten läßt und bei der gleichzeitig eine Trennung Pigment/Farbmittel im System weitgehend ausgeschlossen ist.

Überraschenderweise wurde nun eine Pigmentmischung gefunden, die keine der oben angegebenen Nachteile aufweist. Das erfindungsgemäße Pigmentgemisch besteht aus mindestens zwei Komponenten, wobei Komponente A mit ein oder mehreren Metalloxiden beschichtete SiO₂₋Flakes und Komponente B ein oder mehrere Effektpigmente sind. Durch die Zumischung der Effektpigmente zu den beschichteten SiO₂-Flakes kann den Anwendungssystemen ein Mehrfachflop verliehen werden, der Farbeffekt wird verstärkt und neuartige Farbeffekte werden erzielt.

Gegenstand der Erfindung ist somit ein Pigmentgemisch bestehend aus mindestens zwei Komponenten, die im Verhältnis 10:1 bis 1:10 gemischt sind, wobei Komponente A mit ein oder mehreren Metalloxiden und/oder Metallen beschichtete SiO₂-Flakes und Komponente B Effektpigmente ausgewählt aus der Gruppe der Metalleffektpigmente, beschichteten Eisenoxidplättchen, der beschichteten und unbeschichteten Aluminium-plättchen, der Perlglanzpigmente, mit Metalloxiden beschichtete Al₂O₃₋Flakes, Graphitplättchen, BiOCl oder Glasflakes sind.

Gegenstand der Erfindung sind ebenfalls die Formulierungen, wie z. B. Farben, Lacke, Druckfarben, Kunststoffe, Agrarfolien und kosmetische Formulierungen, die das erfindungsgemäße Pigmentgemisch enthalten.

Die beschichteten SiO₂-Flakes können in jedem Verhältnis mit den Effektpigmenten gemischt werden. Vorzugsweise ist das Verhältnis von Komponente A zu Komponente B 1 : 10 bis 10 :1, insbesondere 3 : 1 bis 5: 1.

Die vorzugsweise nach der WO 93/08237 auf einem endlosen Band hergestellten SiO₂-Flakes basieren auf einer plättchenförmigen, transparenten Matrix und besitzen in der Regel eine Dicke zwischen 0,1 und 5 µm, insbesondere zwischen 0,2 und 2,0 µm. Die Ausdehnung in den beiden anderen Dimensionen beträgt üblicherweise zwischen 1 und 250 µm, vorzugsweise zwischen 2 und 100 µm, und insbesondere zwischen 5 und 40 µm. Die SiO₂-Flakes werden mit ein oder mehreren Metalloxidschichten und/oder Metallschichten versehen. Geeignete Metalloxide oder Metalloxidgemische sind beispielsweise Titandioxid, Zirkonoxid, Zinkoxid, Eisenoxide und/oder Chromoxid, insbesondere TiO₂ und/oder Fe₂O₃. Die Beschichtung der SiO₂-Flakes kann z.B. erfolgen wie in der WO 93/08237 (naßchemische Beschichtung) oder DE-OS-196 14 637 (CVD-Verfahren) beschrieben.

Anstelle der äußeren Metalloxidschicht kann auch eine semitransparente Schicht eines Metalls verwendet werden. Geeignete Metalle dafür sind beispielsweise Cr, Ti, Mo, W, Al, Cu, Ag, Au, oder Ni. Bevorzugte Pigmente weisen folgenden Schichtaufbau auf: SiO₂-Flakes + Metall + SiO₂ + Metalloxid.

Zur Erzielung spezieller Farbeffekte können in die hoch- bzw. niedrigbrechenden Metalloxidschichten zusätzlich noch feinteilige Partikel im Nanometergrößenbereich eingebracht werden. Als geeignet dafür erweisen sich beispielsweise feinteiliges TiO₂ oder feinteiliger Kohlenstoff (Ruß) mit Teilchengrößen im Bereich von 10 - 250 nm. Durch die lichtstreuenden Eigenschaften derartiger Partikel kann gezielt auf Glanz und Deckvermögen Einfluß genommen werden. Vorzugsweise sind die SiO₂-Flakes mit ein oder mehreren Metalloxiden beschichtet.

Als Komponente B für die erfindungsgemäße Pigmentmischung sind alle dem Fachmann im Effektpigmentsektor gängigen Effektpigmente, wie z.B. Metalleffektpigmente, wie Aluminium, Kupfer, Zink, Zinn und ihre Legierungen geeignet. Aluminium- und Goldbronzelegierungen sind bevorzugt zu nennen, insbesondere solche, die eine Partikelgröße von 2 bis 40 µm aufweisen. Bevorzugt enthalten die erfindungsgemäßen Pigmentgemische beschichtetes plättchenförmiges Eisenoxid, Aluminiumflakes oder beschichtete Aluminiumflakes. Derartige Effektpigmente werden von der BASF unter dem Namen Paliocrom®, von den Eckart-Werken unter dem Namen Standard® und von der Firma Flex vertrieben. Weiterhin zu nennen sind Perlglanzpigmente, mit Metalloxiden, wie z.B. TiO₂, Fe₂O₃, beschichtete Al₂O₃-Flakes, Graphitplättchen, BiOCl oder Glasflakes.

Perlglanzpigmente, mit ein oder mehreren Metalloxiden beschichtete Glimmerschuppenpigmente, sind erhältlich z.B. von der Fa. Merck KGaA, Darmstadt, unter dem Handelsnamen Iriodin®. Letztere sind z.B. bekannt aus den deutschen Patenten und Patentanmeldungen 14 67 468, 19 59 998, 20 09 566, 22 14 545, 22 15 191, 22 44 298, 23 13 331, 25 22 572, 31 37 808, 31 37 809, 31 51 343, 31 51 354, 31 51 355, 32 11 602 und 32 53 017. Insbesondere werden mit TiO₂ und/oder Fe₂O₃ beschichtete Glimmerpigmente eingesetzt. Als Schichtsilikat sind sowohl der natürliche als auch der synthetische Glimmer geeignet.

Die erfindungsgemäße Pigmentmischung ist einfach und leicht handzuhaben. Die Pigmentmischung kann durch einfaches Einrühren in das Anwendungssystem eingearbeitet werden. Ein aufwendiges Mahlen und Dispergieren der Pigmente ist nicht erforderlich.

Die erfindungsgemäße Pigmentmischung kann zur Pigmentierung von Lacken, Druckfarben, Kunststoffen, Agrarfolien, Knopfpasten, zur Saatgut-baschichtung, Lebensmitteleinfärbung, Arzneimittelüberzügen oder kosmetischen Formulierungen verwendet werden. Die Konzentration der Pigmentmischung im zu pigmentierendem Anwendungssystem liegt in der Regel zwischen 0,01 und 50 Gew.%, bevorzugt zwischen 0,1 und 5 Gew.% bezogen auf den Gesamtfestkörpergehalt des Systems. Sie ist in der Regel abhängig vom konkreten Anwendungsfall.

Kunststoffe enthaltend das erfindungsgemäße Pigmentgemisch in Mengen von 0,1 bis 50 Gew. %, insbesondere 0,5 bis 7 Gew.%, zeichnen sich häufig durch einen besonderen Sparkle-Effekt aus.

Im Lackbereich, insbesondere im Automobillack, wird das Pigmentgemisch in Mengen von 0,5-10 Gew.% eingesetzt. Das Mischungsverhältnis der beschichteten SiO₂-Flakes mit Komponente B, insbesondere.beschichteten oder unbeschichteten Aluminiumflakes, hängt vom gewünschten Effekt ab. Vorzugsweise werden die SiO₂-Flakes mit Komponente B im Verhältnis von 5 : 1, insbesondere von 3 : 1 eingesetzt.
Im Lack hat die erfindungsgemäße Pigmentmischung den Vorteil, daß der angestrebte Farbflop-Effekt durch eine einschichtige Lackierung (Einschichtsysteme bzw. als Basecoat im 2-Schichtaufbau) erzielt wird. Dieser Farbflop ist auch im diffusen Licht sehr deutlich ausgeprägt. Im Vergleich mit Lackierungen, die ein Interferenzpigment enthalten statt der beschichteten SiO₂-Flakes, zeigen Lackierungen mit der erfindungsgemäßen Pigmentmischung eine deutliche Tiefenwirkung und einen Glitzereffekt sowie einen starken Farbflop.

Bei der Pigmentierung von Bindemittelsystemen, z. B. für Farben und Druckfarben für den Tiefdruck, Offsetdruck oder Siebdruck, haben sich insbesondere Pigmentgemische bestehend aus beschichteten SiO₂-Flakes mit Stapa® - Aluminium- und Goldbronzepasten der Fa. Eckart-Werke - als besonders geeignet erwiesen. Das Pigmentgemisch wird in die Druckfarbe in Mengen von 2-50 Gew.%, vorzugsweise 5-30 Gew.%, und insbesondere 8-15 Gew.% eingearbeitet. Das Mischungsverhältnis von Komponente A zu Komponente B liegt vorzugsweise im Bereich von 1 : 10 bis 10 : 1. Die Druckfarben enthaltend das erfindungsgemäße Pigmentgemisch zeigen reinere Farbtöne und sind aufgrund der guten Werte für die Viskosität besser verdruckbar.

Gegenstand der Erfindung sind ebenfalls Pigmentpräparationen enthaltend beschichtete oder unbeschichtete SiO₂-Flakes, Metalleffektpigmente, Bindemittel und gegebenenfalls Additive, die in Form eines weitgehend lösungsmittelfreien, rieselfähigen Granulats vorliegen. Derartige Granulate enthalten bis zu 95 Gew.% des Pigmentgemisches. Eine Pigmentzubereitung, bei der das erfindungsgemäße Pigmentgemisch mit einem Bindemittel und mit Wasser oder einem organischen Lösemittel und gegebenenfalls Additiven angepastet wird, und die Paste nachfolgend getrocknet und in eine kompakte Teilchenform, z.B. in Granulate, Pellets, Briketts, Masterbatch, Tabletten, gebracht wird, ist insbesondere als Vorprodukt von Druckfarben geeignet.

Gegenstand der Erfindung sind somit auch Formulierungen enthaltend das erfindungsgemäße Pigmentgemisch.

Die nachfolgenden Beispiele sollen die Erfindung erläutern, ohne sie jedoch zu begrenzen.

### Beispiele

### Beispiel 1: Lack

Formulierungen bestehend aus (Angeben in Gew.%)

| | |
|---|---|
| 2,50 % | mit Fe₂O₃ beschichteten SiO₂-Flakes mit einer Teilchengröße von 5 - 40 µm (Fa. Merck KGaA) |
| 1,50 % | Monastralgrün® 6Y spez. (Fa. Zeneca) |
| 0,50 % | Cappoxytgelb® 4214 (Fa. Cappelle) |
| 0,03 % | Farbruß FW 200 (Fa. Degussa) |
| 0,40 % | Dollaraluminium Alpate 7620 NS (Fa. Alcan Toyo Europe) |

Rest: Basislack mit 19 % Festkörpergehalt (Acrylat-Melamin) und Verdünnermischung

### Beispiel 2: Tiefdruck

Druckfarbe bestehend aus

| | |
|---|---|
| 70 g | Bindemittel der Gebrüder Schmidt 95MB011 auf Nitrocellulosebasis mit ca. 20 % Feststoffgehalt |
| 30 g | Pigment 15 g Cromal IV (Fa. Eckart) Al 14-18 µm 15 g mit Fe₂O₃ beschichtete SiO₂-Flakes der Teilchengröße 5-40 µm |
| 30 g | 1-Ethoxy-2-propanol |

### Beispiel 3: Kunststoff

1 kg Polystyrolgranulat werden in einem Taumelmischer mit 5 g Haftmittel gleichmäßig benetzt. Dazu werden dann 35 g Fe₂O₃ beschichtete SiO₂₋Flakes der Teilchengröße von 5-40 µm und 7 g Iriodin® 121 mit TiO₂ beschichtetes Glimmerpigment der Fa. Merck KGaA, Darmstadt, BRD mit einer Teilchengröße von 5-20 µm zugegeben und 2 min. lang gemischt

Dieses Granulat wird auf einer Spritzgießmaschine unter üblichen Bedingungen zu Stufenplättchen mit den Maßen 4 x 3 x 0,5 cm verarbeitet. Die Stufenplättchen zeichnen sich durch ihren Glanz aus.

### Beispiel 4: Lidschatten

### Phase A

| | |
|---|---|
| 15,00 % | mit TiO₂ beschichtete SiO₂-Flakes der Teilchengröße 5-40 µm (Fa. Merck KGaA) |
| 15,00 % | Timiron Super Blue (mit TiO₂ beschichteter Glimmer der Teilchengröße 10-60 µm der Fa. Merck KGaA) |
| 49.50 % | Talc |
| 7,50 % | Solanum Tuberosum (Potato Starch) |
| 2,50 % | Magnesium Stearate |

### Phase B

| | |
|---|---|
| 9,14 % | Isopropyl Stearate |
| 0,53 % | Cetyl Palmitate |
| 0,53 % | Petrolatum |
| 0,21 % | Parfüm |
| 0,11 % | Konservierung |

Die Bestandteile der Phase A werden zusammengegeben und vorgemischt. Anschließend wird die Pudermischung unter Rühren tropfenweise mit der geschmolzenen Phase B versetzt. Die Puder werden bei 40 - 50 bar gepreßt.

### Beispiel 5: Duschgel

### Phase A

| | |
|---|---|
| 0,10 % | mit TiO₂ beschichtete SiO₂-Flakes der Teilchengröße 5-40 µm (Fa. Merck KGaA) |
| 0,10 % | Timiron Super Blue (mit TiO₂ beschichteter Glimmer der Teilchengröße 10-60 µm der Fa. Merck KGaA) |
| 0,75 % | Xanthan Gum |
| ad 100,00 % | Aqua |

### Phase B

| | |
|---|---|
| 20,00 % | Decyl Glycoside |
| 6,65 % | Texapon ASV |
| | Sodium Laureth Sulfate |
| | Magnesium Laureth Sulfate |
| | Sodium Laureth 8-Sulfate |
| | Magnesium Laureth 8-Sulfate |
| | Sodium Oleth Sulfate |
| | Magnesium Oleth Sulfate |
| 0,20 % | Konservierung |
| 0,05 % | Parfum |

### Phase C

| | |
|---|---|
| 0,15 % | Citric Acid |
| 10,00 % | Aqua |

Für Phase A wird das Pigment in das Wasser eingerührt. Xanthan Gum wird unter Rühren langsam eingestreut und gerührt, bis es gelöst ist. Die Phasen B und C werden nacheinander hinzugefügt und dabei langsam gerührt, bis alles homogen verteilt ist.

## Patentansprüche

1. Pigmentgemisch bestehend aus mindestens zwei Komponenten, die im Verhältnis 10:1 bis 1:10 gemischt sind, wobei Komponente A mit ein oder mehreren Metalloxiden und/oder Metallen beschichtete SiO₂-Flakes und Komponente B Effektpigmente ausgewählt aus der Gruppe der Metalleffektpigmente, beschichteten Eisenoxidplättchen, der beschichteten oder unbeschichteten Aluminiumplättchen, der Periglanzpigmente, mit Metalloxiden beschichtete Al₂O₃-Flakes, Graphitplättchen, BiOCl oder Glasflakes sind.

2. Pigmentgemisch nach Anspruch 1, **dadurch gekennzeichnet, dass** die SiO₂-Flakes auf einer plättchenförmigen, transparenten Matrix basieren und eine Dicke zwischen 0,1 und 5 µm besitzen.

3. Pigmentgemisch nach Anspruch 1 oder 2, **dadurch gekennzeichnet, dass** die Metalloxide oder Metalloxidgemische in Komponente A aus Titandioxid, Zirkonoxid, Zinkoxid, Eisenoxiden und/oder Chromoxid sind.

4. Pigmentgemisch nach einem der Ansprüche 1 bis 3, **dadurch gekennzeichnet, dass** Komponente A mit TiO₂ und/oder Fe₂O₃ beschichtete SiO₂-Flakes sind.

5. Pigmentgemisch nach einem der Ansprüche 1 bis 4, **dadurch gekennzeichnet, dass** Komponente A SiO₂-Flakes + Metall + SiO₂ + Metalloxid als Schichtaufbau enthält.

6. Pigmentgemisch nach einem der Ansprüche 1 bis 5, **dadurch gekennzeichnet, dass** in die hoch- bzw. niedrigbrechenden Metalloxidschichten zusätzlich noch feinteilige Partikel im Nanometergrößenbereich eingebracht werden.

7. Pigmentgemisch nach einem der Ansprüche 1 bis 6, **dadurch gekennzeichnet, dass** Komponente B mit ein oder mehreren Metalloxiden beschichtete Metallplättchen, Graphitplättchen, Aluminiumplättchen, Schichtsilikate, Al₂O₃-Flakes, Fe₂O₃-Flakes, TiO₂-Flakes, Glas- und/oder keramische Plättchen sind.

8. Pigmentgemisch nach einem der Ansprüche 1 bis 7, **dadurch gekennzeichnet, dass** die Metalleffektpigmente in Komponente B ausgewählt sind aus Aluminium, Kupfer, Zink, Zinn und deren Legierungen.

9. Verwendung des Pigmentgemisches nach einem der Ansprüche 1 bis 8 in Farben, Lacken, Druckfarben, Pulverlacken, Masterbatches, Kunststoffen, zur Saatguteinfärbung, zur Lebensmitteleinfärbung, in Knopfpasten, in Arzneimittelüberzügen und in kosmetischen Formulierungen.

10. Formulierungen enthaltend ein Pigmentgemisch nach einem der Ansprüche 1 bis 8.

11. Formulierungen nach Anspruch 10, **dadurch gekennzeichnet, dass** die Konzentration des Pigmentgemisches im Anwendungssystem zwischen 0,01 und 50 Gew.% bezogen auf den Gesamtfestkörper gehalt des Systems liegt.

12. Pigmentzubereitung enthaltend ein Pigmentgemisch nach einem der Ansprüche 1 bis 8 und ein Bindemittel, **dadurch gekennzeichnet, dass** sie in Form eines weitgehend lösemittelfreien, rieselfähigen Granulats vorliegt und bis zu 95 % des Pigmentgemisches enthält.

## Claims

1. Pigment mixture consisting of at least two components which are mixed in the ratio 10:1 to 1:10, where component A comprises SiO₂ flakes coated with one or more metal oxides and/or metals and component B comprises effect pigments selected from the group of the metal-effect pigments, coated iron oxide platelets, coated or uncoated aluminium platelets, pearlescent pigments, metal oxide-coated Al₂O₃ flakes, graphite platelets, BiOCl or glass flakes.

2. Pigment mixture according to Claim 1, **characterised in that** the SiO₂ flakes are based on a platelet-shaped, transparent matrix and have a thickness between 0.1 and 5 µm.

3. Pigment mixture according to Claim 1 or 2, **characterised in that** the metal oxides or metal-oxide mixtures in component A comprise titanium dioxide, zirconium oxide, zinc oxide, iron oxides and/or chromium oxide.

4. Pigment mixture according to one of Claims 1 to 3, **characterised in that** component A comprises TiO₂- and/or Fe₂O₃-coated SiO₂ flakes.

5. Pigment mixture according to one of Claims 1 to 4, **characterised in that** component A comprises SiO₂ flakes + metal + SiO₂ + metal oxide as layer structure.

6. Pigment mixture according to one of Claims 1 to 5, **characterised in that** finely divided particles in the nanometre size range are additionally introduced into the high- or low-refracting metal-oxide layers.

7. Pigment mixture according to one of Claims 1 to 6, **characterised in that** component B comprises metal platelets, graphite platelets, aluminium platelets, phyllosilicates, Al₂O₃ flakes, Fe₂O₃ flakes, TiO₂ flakes, glass platelets and/or ceramic platelets coated with one or more metal oxides.

8. Pigment mixture according to one of Claims 1 to 7, **characterised in that** the metal-effect pigments in component B are selected from aluminium, copper, zinc, tin and alloys thereof.

9. Use of the pigment mixture according to one of Claims 1 to 8 in paints, coatings, printing inks, powder coatings, masterbatches, plastics, for colouring seed, for colouring foods, in button pastes, in medicament coatings and in cosmetic formulations.

10. Formulations comprising a pigment mixture according to one of Claims 1 to 8.

11. Formulations according to Claim 10, **characterised in that** they are present in the application system between 0.01 and 50% by weight, based on the total solids content of the system.

12. Pigment composition comprising a pigment mixture according to one of Claims 1 to 8 and a binder, **characterised in that** it is in the form of substantially solvent-free, free-flowing granules and comprises up to 95% of the pigment mixture.

## Revendications

1. Mélange de pigments constitué par au moins deux composants qui sont mélangés selon le rapport 10:1 à 1:10, où un composant A comprend des flocons de SiO₂ revêtus d'un ou de plusieurs oxydes métalliques et/ou de métaux et un composant B comprend des pigments d'effet choisis parmi le groupe des pigments d'effet métallique, des plaquettes d'oxyde de fer revêtues, des plaquettes d'aluminium revêtues ou non revêtues, des pigments perlés, des flocons d'Al₂O₃ revêtus d'oxyde métallique, des plaquettes en graphite, des flocons de BiOCl ou de verre.

2. Mélange de pigments selon la revendication 1, **caractérisé en ce que** les flocons de SiO₂ sont basés sur une matrice transparente en forme de plaquette, et présentent une épaisseur entre 0,1 et 5 um.

3. Mélange de pigments selon la revendication 1 ou 2, **caractérisé en ce que** les oxydes métalliques ou les mélanges d'oxydes métalliques dans le composant A comprennent dioxyde de titane, oxyde de zirconium, oxyde de zinc, oxydes de fer et/ou oxyde de chrome.

4. Mélange de pigments selon l'une des revendications 1 à 3, **caractérisé en ce que** le composant A comprend des flocons de SiO₂ revêtus de TiO₂ et/ou de Fe₂O₃.

5. Mélange de pigments selon l'une des revendications 1 à 4, **caractérisé en ce que** le composant A comprend des flocons de SiO₂ + métal + SiO₂ + oxyde métallique en tant que structure en couches.

6. Mélange de pigments selon l'une des revendications 1 à 5, **caractérisé en ce que** des particules finement divisées dans la plage de dimensions des nanomètres sont additionnellement introduites dans les couches d'oxyde métallique haute ou basse réfraction.

7. Mélange de pigments selon l'une des revendications 1 à 6, **caractérisé en ce que** le composant B comprend des plaquettes métalliques, des plaquettes en graphite, des plaquettes en aluminium, des phyllosilicates, des flocons de Al₂O₃, des flocons de Fe₂O₃, des flocons de TiO₂, des plaquettes en verre et/ou des plaquettes en céramique revêtues d'un ou de plusieurs oxydes métalliques.

8. Mélange de pigments selon l'une des revendications 1 à 7, **caractérisé en ce que** les pigments d'effet métallique dans le composant B sont choisis parmi aluminium, cuivre, zinc, étain et leurs alliages.

9. Utilisation du mélange de pigments selon l'une des revendications 1 à 8 dans des peintures, des revêtements, des encres d'impression, des revêtements pulvérulents, des mélanges maîtres, des matières plastiques, pour colorer des graines, pour colorer des aliments, dans des pâtes pour les boutons, dans des enrobages de médicament et dans des formulations cosmétiques.

10. Formulations comprenant un mélange de pigments selon l'une des revendications 1 à 8.

11. Formulations selon la revendication 10, **caractérisées en ce qu'**elles sont présentes dans le système d'application entre 0,01 et 50% en poids, sur la base de la teneur totale en solides du système.

12. Composition de pigments comprenant un mélange de pigments selon l'une des revendications 1 à 8 et un liant, **caractérisée en ce qu'**elle est sous la forme de granules sensiblement exempts de solvant fluant librement et comprend jusqu'à 95% du mélange de pigments.
